# EUROPÄISCHE PATENTSCHRIFT

(11) **EP 1 845 934 B1**
(45) Veröffentlichungstag und Bekanntmachung des Hinweises auf die Patenterteilung: **14.05.2008**
(21) Anmeldenummer: 06701288.0
(22) Anmeldetag: 25.01.2006
(51) Int. Cl.: A61K 8/49, A61K 8/34, A61Q 5/00, A61Q 5/02, A61Q 19/10

(54) **KONSERVIERUNGSMITTEL**
PRESERVATIVES
AGENTS DE CONSERVATION

(30) Priorität: 03.02.2005 DE 102005005009; 01.07.2005 DE 102005030762
(43) Veröffentlichungstag der Anmeldung: 24.10.2007
(73) Patentinhaber: Clariant Produkte (Deutschland) GmbH, 65929 Frankfurt am Main (DE)
(72) Erfinder: KLUG, Peter, 63762 Grossostheim (DE); KLEIN, Sonja, 65439 Flörsheim (DE); SIMSCH, Waltraud, 65779 Kelkheim (DE)
(74) Vertreter: Hütter, Klaus
(86) Internationale Anmeldenummer: PCT/EP2006/000627
(87) Internationale Veröffentlichungsnummer: WO 2006/081969

(56) Entgegenhaltungen:
- EP-A- 0 241 918
- WO-A-00/06106
- WO-A-01/06997
- FR-A- 2 685 867
- US-A- 6 121 254
- US-A1- 2004 234 482
- DATABASE WPI Section Ch, Week 199824 Derwent Publications Ltd., London, GB; Class B03, AN 1998-269954 XP002377202 & RU 2 093 141 C1 (IRIKOV V A) 20. Oktober 1997 (1997-10-20) & RU 2 093 141 C1 (IRIKOV VITALIJ A) 20. Oktober 1997 (1997-10-20)
- BACH M ET AL: "KONSERVIERUNGSMITTEL UND IHRE PRAKTISCHE ANWENDUNG IN KOSMETISCHEN PRODUKTEN" SOFW-JOURNAL SEIFEN, OELE, FETTE, WACHSE, VERLAG FUR CHEMISCHE INDUSTRIE, AUGSBURG, DE, Bd. 116, Nr. 9, 13. Juni 1990 (1990-06-13), Seiten 345-356, XP000134744 ISSN: 0942-7694

## Beschreibung

Die vorliegende Erfindung betrifft klare und flüssige Mittel, enthaltend 1-Hydroxy-4-methyl-6-alkyl-2(1 H)-pyridone wie z.B. 1-Hydroxy-4-methyl-6-(2,4,4-trimethylpentyl)-2(1 H)-pyridon und/oder ein oder mehrere Salze davon und mindestens einen Alkohol, enthaltend eine oder mehrere aromatische Gruppen, zum Konservieren von Zubereitungen.

Die Verwendung von Piroctone Olamine (2-Aminoethanol-Salz von 1-Hydroxy-4-methyl-6-(2,4,4-trimethylpentyl)-2(1H)-pyridon, Handelsprodukt ist Octopirox^{®}) und auch von 2-Phenoxyethanol oder Benzylalkohol zum Konservieren kosmetischer Mittel ist bekannt. In "Seifen - Öle - Fette - Wachse", 116, Nr. 9/ 1990, S. 345-356 wird beschrieben, dass Pirocton Olamin in Shampoos eingesetzt werden kann, wobei für eine ausreichende Konservierung eine Gewichtsmenge an Piroctone Olamine von 0,5 bis 1 % erforderlich ist. Für O/W-Emulsionen und W/O-Emulsionen ist die antimikrobielle Wirkung von Piroctone Olamine bei der Einsatzmenge von 0,5 Gew.-% unzureichend.

Gemäß der europäischen Kosmetik-Richtlinie 76/768/EEC, ANNEX 6 darf Piroctone Olamine in Rinse-off Produkten in Konzentrationen von maximal 1 Gew.-%, in allen anderen kosmetischen Produkten in Konzentrationen von maximal 0,5 Gew.-%, eingesetzt werden.

Die Einarbeitbarkeit von Piroctone Olamine in Kosmetika ist durch die geringe Löslichkeit der Wirksubstanz in Wasser und nur mäßige Löslichkeit in Öl eingeschränkt.

Es bestand die Aufgabe, hochwirksame Konservierungsmittel für kosmetische, pharmazeutische und dermatologische Zubereitungen zur Verfügung zu stellen, die dermatologisch und toxikologisch unbedenklich sind, mit wässrigen Systemen und mit Ölsystemen gut verträglich sind, ein klares visuelles Erscheinungsbild haben, leicht verarbeitbar sind und Temperatur- und Lagerbeständigkeit aufweisen.

Es wurde überraschend gefunden, dass diese Aufgabe gelöst wird durch klare, flüssige Konservierungsmittel, enthaltend
a) ein oder mehrere 1-Hydroxy-4-methyl-6-alkyl-2(1H)-pyridone und/oder ein oder mehrere Salze davon wobei Alkyl eine lineare, verzweigte oder cyclische Alkylgruppe mit 1 bis 12 Kohlenstoffatomen bedeutet und
b) einen oder mehrere Alkohole, enthaltend ein oder mehrere aromatische Gruppen.

Die antimikrobielle Wirkung, sowohl von 1-Hydroxy-4-methyl-6-alkyl-2(1 H)-pyridonen wie z.B. Piroctone Olamine als auch die der Alkohole, enthaltend eine oder mehrere aromatische Gruppen, wird dabei synergistisch verstärkt. Die Einsatzkonzentrationen dieser antimikrobiellen Wirksubstanzen in kosmetischen, dermatologischen und pharmazeutischen Produkten können dadurch signifikant erniedrigt werden.

Es wurde weiter gefunden, dass die erfindungsgemäßen Konservierungsmittel geeignet sind zum Einsatz in rinse-off, aber auch in leave-on Produkten, insbesondere in Emulsionen, vorzugsweise in O/W-Emulsionen oder W/O-Emulsionen, und bei effektiven Einsatzkonzentrationen des Piroctone Olamines von unterhalb 0,1 Gew.-% eine ausreichende Konservierung der Produkte bewirken.

Vorteilhaft ist ebenfalls das klare Erscheinungsbild der flüssigen Konservierungsmittel und dessen gute Formulierbarkeit.

Gegenstand der Erfindung sind klare, flüssige Konservierungsmittel, enthaltend
a) ein oder mehrere 1-Hydroxy-4-methyl-6-alkyl-2(1H)-pyridone und/oder ein oder mehrere Salze davon wobei Alkyl eine lineare, verzweigte oder cyclische Alkylgruppe mit 1 bis 12 Kohlenstoffatomen, vorzugsweise 2,4,4-Trimethylpentyl oder Cyclohexyl, bedeutet und
b) einen oder mehrere Alkohole, enthaltend ein oder mehrere aromatische Gruppen.

In einer bevorzugten Ausführungsform der Erfindung enthalten die erfindungsgemäßen Konservierungsmittel
a) 1-Hydroxy-4-methyl-6-(2,4,4-trimethylpentyl)-2(1H)-pyridon und/oder ein oder mehrere Salze davon und
b) einen oder mehrere Alkohole, enthaltend eine oder mehrere, vorzugsweise eine, aromatische Gruppe.

In einer besonders bevorzugten Ausführungsform enthalten die erfindungsgemäßen Konservierungsmittel
a) 1-Hydroxy-4-methyl-6-(2,4,4-trimethylpentyl)-2(1H)-pyridon und/oder ein oder mehrere Salze davon in Gewichtsmengen von 0,1 bis 20 %, bevorzugt 1,1 bis 10 %, besonders bevorzugt 1,5 bis 7 % und insbesondere bevorzugt 5 % und
b) einen oder mehrere Alkohole, enthaltend eine oder mehrere aromatische Gruppen in Gewichtsmengen von 35 bis 99,9 und vorzugsweise von 80 bis 99,9%.

In einer weiteren besonders bevorzugten Ausführungsform enthalten die erfindungsgemäßen Konservierungsmittel
a) 1-Hydroxy-4-methyl-6-(2,4,4-trimethylpentyl)-2(1 H)-pyridon und/oder ein oder mehrere Salze davon,
b) einen oder mehrere Alkohole, enthaltend ein oder mehrere aromatische Gruppen,
c) Wasser,
d) optional einen oder mehrere weitere antimikrobielle Wirkstoffe,
e) optional ein oder mehrere Hydrotrope und
f) optional ein oder mehrere weitere Additive.

Die erfindungsgemäßen Konservierungsmittel, enthaltend ein oder mehrere 1-Hydroxy-4-methyl-6-alkyl-2(1 H)-pyridone wie z. B. 1-Hydroxy-4-methyl-6-(2,4,4-trimethylpentyl)-2(1 H)-pyridon und/oder ein oder mehrere Salze davon, einen oder mehrere Alkohole, enthaltend ein oder mehrere aromatische Gruppen und Wasser sind klare Flüssigkeiten, die auch nach monatelanger Lagerung keine Kristallisationstendenzen zeigen.

In einer weiteren besonders bevorzugten Ausführungsform, enthalten die erfindungsgemäßen Konservierungsmittel
a) 1-Hydroxy-4-methyl-6-(2,4,4-trimethylpentyl)-2(1H)-pyridon und/oder ein oder mehrere Salze davon in Gewichtsmengen von 0,1 bis 20 %, bevorzugt 1,1 bis 10 %, besonders bevorzugt 1,5 bis 7 % und insbesondere bevorzugt 5 %,
b) einen oder mehrere Alkohole, enthaltend eine oder mehrere aromatische Gruppen in Gewichtsmengen von 35 bis 99,8 und bevorzugt von 59,8 bis 99,8 % und
c) Wasser in Gewichtsmengen von 0,1 bis 35 %, bevorzugt 0,1 bis 20 %, besonders bevorzugt 0,5 bis 5 % und insbesondere bevorzugt 1 bis 3 %.

Die erfindungsgemäßen Konservierungsmittel enthalten ein oder mehrere 1-Hydroxy-4-methyl-6-alkyl-2(1H)-pyridone wie z.B. 1-Hydroxy-4-methyl-6-(2,4,4-trimethylpentyl)-2(1H)-pyridon und/oder ein oder mehrere Salze davon, wobei es sich bei den Salzen um Li⁺-, Na⁺-, K⁺-, Mg⁺⁺-, Ca⁺⁺-, Zn⁺⁺-, Al⁺⁺⁺-, NH₄⁺-, Monoalkylammonium-, Dialkylammonium-, Trialkylammonium- und/oder Tetraalkylammoniumsalze, wobei die Alkylsubstituenten der Amine unabhängig voneinander (C₁-C₂₂)-Alkylreste oder (C₂-C₁₀)-Hydroxyalkylreste sein können, handeln kann.

Unter den genannten Salzen sind die Monoalkylammonium-, Dialkylammonium-, Trialkylammonium- und Tetraalkylammoniumsalze bevorzugt.

Besonders bevorzugt enthalten die erfindungsgemäßen Konservierungsmittel das Monoethanolamin-Salz von 1-Hydroxy-4-methyl-6-(2,4,4-trimethylpentyl)-2(1 H)-pyridon (Octopirox^{®}, Clariant GmbH).

Die in den erfindungsgemäßen Konservierungsmitteln enthaltenen Alkohole enthaltend ein oder mehrere aromatische Gruppen sind vorzugsweise ausgewählt aus 2-Phenoxyethanol, 1-Phenoxy-2-propanol und Benzylalkohol. Unter diesen Alkoholen ist wiederum 2-Phenoxyethanol besonders bevorzugt.

In einer weiteren besonders bevorzugten Ausführungsform enthalten die erfindungsgemäßen Konservierungsmittel ein oder mehrere weitere antimikrobielle Wirkstoffe, vorzugsweise in den Konzentrationen von 1 bis 25 Gew.-%, besonders bevorzugt von 5 bis 25 Gew.-% und insbesondere bevorzugt von 10 bis 15 Gew.-%. Die weiteren antimikrobiellen Wirkstoffe sind vorzugsweise ausgewählt aus Parabenen, wie beispielsweise Methylparaben, Ethylparaben, Propylparaben, Isopropylparaben, Butylparaben, Isobutylparaben, Natrium-Methylparaben, Natrium-Ethylparaben, Natrium-Propylparaben Natriumisobutylparaben, Natriumisopropylparaben oder Natrium-Butylparaben, Imidazolidinyl Harnstoff, Diazolidinyl Harnstoff, Iodopropynyl Butylcarbamat, 2-Bromo-2-Nitropropan-1,3-diol, Sorbinsäure, Kaliumsorbat, Cetyltrimethylammoniumchlorid, Cetylpyridiniumchlorid, Benzethoniumchlorid, Diisobutylethoxyethyl-dimethylbenzylammoniumchlorid, Düsobutyl-phenoxyethoxy-ethyl-dimethylbenzyl-ammoniumchlorid, N-Alkyl-N,N-dimethyl-benzylammoniumchlorid, -bromid, -saccharinat, Trimethylammoniumchlorid, Natriumaluminiumchlorohydroxylactat, Triethylcitrat, Tricetylmethylammoniumchlorid, 2,4,4'-Trichloro-2'-hydroxydiphenylether (Triclosan), 3,4,4'-Trichlorocarbanilid (Triclocarban), Diaminoalkylamid, beispielsweise L-Lysinhexadecylamid, DMDM Hydantoin, Natrium Hydroxymethylglycinat, Benzoesäure, Propionsäure, Salicylsäure, 2,4-Hexandiensäure, 2-Hydroxybiphenyl, Chlorbutanulum, 3-Acetyl-methyl-2,4-(3H)pyrandion, Ameisensäure, Undecenylsäure, 5-Amino-1,3-bis-(2-ethylhexyl)-5-methyl-hexahydropyrimidin, 2-Bromo-2-nitro-1,3-propandiol, 2,4-Dichlorbenzylalkohol, N-(4-Chlorphenyl-N'-(3,4-dichlorphenyl)harnstoff, 2,4,4'-Trichlor-2'-hydroxy-diphenylether, Poly(hexamethylendiguanid)-hydrochlorid, 1,2-Dibrom-2,4-dicyanobutan, 4,4-Dimethyl-1,3-oxazolidin, Natriumbenzoat, Methylisothiazolinon, Methylchloroisothiazolinon und Methylisothiazolinon im molaren Verhältnis von 3:1, Chlorooxyenol, Citratschwermetallsalzen, Silberchlorid, Salicylaten wie z.B. Natriumsalicylat, Piroctose, insbesondere Zinksalzen, Pyrithionen und deren Schwermetallsalzen, insbesondere Zinkpyrithion, Zinkphenolsulfat, Farnesol, Ketoconazol, Oxiconazol, Terbinafin, Bifonazole, Butoconazole, Cloconazole, Clotrimazole, Econazole, Enilconazole, Fenticonazole, Isoconazole, Miconazole, Sulconazole, Tioconazole Fluconazole, Itraconazole, Terconazole, Naftifine und Terbinafine und Kombinationen dieser Wirksubstanzen.

In einer insbesondere bevorzugten Ausführungsform enthalten die erfindungsgemäßen Konservierungsmittel ein oder mehrere weitere antimikrobielle Wirkstoffe ausgewählt aus Parabenen, vorzugsweise Methylparaben, Ethylparaben, Propylparaben, Butylparaben,'lsobutylparaben, Natrium-Methylparaben, Natrium-Ethylparaben, Natrium-Propylparaben Natriumisobutylparaben und/oder Natrium-Butylparaben, organischen Säuren, vorzugsweise Sorbinsäure, Kaliumsorbat, Salicylsäure, Natriumsalicylat, Benzoesäure und/oder Natriumbenzoat, Formaldehydabspaltern, vorzugsweise Imidazolidinyl Harnstoff, Diazolidinyl Harnstoff, DMDM Hydantoin und/oder Natrium Hydroxymethylglycinat und halogenierten Konservierungsstoffen, vorzugsweise Iodopropynyl Butylcarbamat und/oder 2-Bromo-2-Nitropropan-1,3-diol.

In einer außerordentlich bevorzugten Ausführungsform enthalten die erfindungsgemäßen Konservierungsmittel
I) Aminoethanolsalz von 1-Hydroxy-4-methyl-6-(2,4,4-trimethylpentyl)-2(1 H)-pyridon, 2-Phenoxyethanol und Methylparaben oder
II) Aminoethanolsalz von 1-Hydroxy-4-methyl-6-(2,4,4-trimethylpentyl)-2(1 H)-pyridon, 2-Phenoxyethanol und Natriumbenzoat oder
III) Aminoethanolsalz von 1-Hydroxy-4-methyl-6-(2,4,4-trimethylpentyl)-2(1H)-pyridon, 2-Phenoxyethanol und Benzoesäure oder
IV) Aminoethanolsalz von 1-Hydroxy-4-methyl-6-(2,4,4-trimethylpentyl)-2(1 H)-pyridon, 2-Phenoxyethanol und Kaliumsorbat oder
V) Aminoethanolsalz von 1-Hydroxy-4-methyl-6-(2,4,4-trimethylpentyl)2(1H)-pyridon, 2-Phenoxyethanol und Sorbinsäure oder
VI) Aminoethanolsalz von 1-Hydroxy-4-methyl-6-(2,4,4-trimethylpentyl)-2(1 H)-pyridon, 2-Phenoxyethanol und Natriumsalicylat oder
VII) Aminoethanolsalz von 1-Hydroxy-4-methyl-6-(2,4,4-trimethylpentyl)-2(1H)-pyridon, 2-Phenoxyethanol und Salicylsäure.

In dem Fall, dass die erfindungsgemäßen Konservierungsmittel ein oder mehrere antimikrobielle Wirkstoffe ausgewählt aus Salzen von organischen Säuren enthalten, enthalten die erfindungsgemäßen Konservierungsmittel vorzugsweise von 10 bis 35 Gew.-% Wasser.

In einer weiteren besonders bevorzugten Ausführungsform enthalten die erfindungsgemäßen Konservierungsmittel ein oder mehrere Antioxidantien. Die Antioxidantien sind vorzugsweise ausgewählt aus Superoxid-Dismutase, Tocopherol (Vitamin E), Ascorbinsäure (Vitamin C), Aminosäuren (z.B. Glycin, Histidin, Tyrosin, Tryptophan) und deren Derivaten, Imidazolen (z.B. Urocaninsäure) und deren Derivaten, Peptiden wie D,L-Carnosin, D-Carnosin, L-Camosin und deren Derivaten (z.B. Anserin), Carotinoiden, Carotinen (z.B. α-Carotin, β-Carotin, Lycopin) und deren Derivaten, Chlorogensäure und deren Derivaten, Liponsäure und deren Derivaten (z.B. Dihydroliponsäure), Aurothioglucose, Propylthiouracil und anderen Thiolen (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salzen, Dilaurylthiodipropionat, Distearylthiodipropionat, Thiodipropionsäure und deren Derivaten (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Apfelsäure), Huminsäure, Gallensäure, Gallenextrakten, Bilirubin, Biliverdin, EDTA, EGTA und deren Derivaten, ungesättigten Fettsäuren und deren Derivaten (z.B. γ-Linolensäure, Linolsäure, Ölsäure), Folsäure und deren Derivaten, Ubichinon und Ubichinol und deren Derivaten, Vitamin C und Derivaten (z.B. Ascorbylpalmitat, Mg-Ascorbylphosphat, Ascorbylacetat), Tocopherolen und Derivaten (z.B. Vitamin-E-acetat), Vitamin A und Derivaten (Vitamin-A-palmitat), Koniferylbenzoat des Benzoeharzes, Rutinsäure und deren Derivaten, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivaten, Mannose und deren Derivaten, Zink und dessen Derivaten (z.B. ZnO, ZnSO₄) Selen und dessen Derivaten (z.B. Selenmethionin), Stilbenen und deren Derivaten (z.B. Stilbenoxid, trans-Stilbenoxid) und Superoxid-Dismutase und erfindungsgemäß geeigneten Derivaten (Salze, Ester, Ether, Zucker, Nukleotide, Nukleoside, Peptide und Lipide) dieser genannten Stoffe.

Besonders vorteilhaft im Sinne der vorliegenden Erfindung können öllösliche Antioxidantien eingesetzt werden.

Außerordentlich bevorzugt enthalten die erfindungsgemäßen Konservierungsmittel ein oder mehrere Antioxidantien ausgewählt aus Tocopherylacetat, BHT (Butylhydroxytoluol) und EDTA.

Die Menge des einen oder der mehreren Antioxidantien in den erfindungsgemäßen Konservierungsmitteln beträgt vorzugsweise 0,001 bis 30 Gew.-%, besonders bevorzugt 0,05 bis 20 Gew.-% und insbesondere bevorzugt 0,1 bis 5 Gew.-%.

In einer weiteren besonders bevorzugten Ausführungsform enthalten die erfindungsgemäßen Konservierungsmittel ein oder mehrere Hydrotrope. Die Hydrotrope sind vorzugsweise ausgewählt aus Xylol-, Toluol- und Cumolsulfonat. Cumolsulfonat ist besonders bevorzugt.

Der Gewichtsanteil des einen oder der mehreren Hydrotrope in den erfindungsgemäßen Mitteln liegt vorzugsweise im Bereich von 1 bis 15 Gew.-%, besonders bevorzugt 4 bis 10 Gew.-% und insbesondere bevorzugt 6 bis 8 Gew.-%, bezogen auf die fertigen Mittel.

In einer weiteren besonders bevorzugten Ausführungsform enthalten die erfindungsgemäßen Konservierungsmittel ein oder mehrere Solubilisatoren, vorzugsweise in Mengen von 1 bis 20 Gew.-%.

Geeignet als Solubilisatoren sind prinzipiell alle ein- oder mehrwertigen Alkohole und ethoxylierten Alkohole, wie z.B. Ethanol, Propanol, Isopropanol, n-Butanol, iso-Butanol, Glycerin und deren Mischungen, Glykole, beispielsweise Triethylenglykol, Butylenglykol, 1,2-Propylenglykol, Polyethylenglykole mit einer relativen Molekülmasse unter 2000, insbesondere mit einer relativen Molekülmasse zwischen 200 und 600, Diole, beispielsweise Pentandiol, 1,5-Pentandiol, 1,6-Hexandiol, Triacetin (Glycerintriacetat), 1-Methoxy-2-propanol und PEG-4-Laurat (Polyethylenglykol-4-Laurat).

Insbesondere bevorzugt enthalten die erfindungsgemäßen Konservierungsmittel ein oder mehrere Solubilisatoren ausgewählt aus Triethylenglykol, Butylenglykol und 1,2-Propylenglykol.

In einer weiteren besonders bevorzugten Ausführungsform bestehen die erfindungsgemäßen Konservierungsmittel aus
a) 1-Hydroxy-4-methyl-6-(2,4,4-trimethylpentyl)-2(1H)-pyridon und/oder ein oder mehreren Salzen davon und
b) einem oder mehreren Alkoholen, enthaltend eine oder mehrere aromatische Gruppen.

In einer weiteren besonders bevorzugten Ausführungsform bestehen die erfindungsgemäßen Konservierungsmittel aus
a) 1-Hydroxy-4-methyl-6-(2,4,4-trimethylpentyl)-2(1H)-pyridon und/oder ein oder mehreren Salzen davon,
b) einem oder mehreren Alkoholen, enthaltend eine oder mehrere aromatische Gruppen und
c) Wasser.

Die erfindungsgemäßen Konservierungsmittel sind in vorteilhafter Weise geeignet zum Konservieren dermatologischer, kosmetischer und pharmazeutischer Produkte.

Weiterer Gegenstand der Erfindung ist daher die Verwendung eines erfindungsgemäßen Mittels zum Konservieren von dermatologischen, kosmetischen und pharmazeutischen Produkten.

Unter den dermatologischen, kosmetischen und pharmazeutischen. Produkten werden im Rahmen der vorliegenden Erfindung z.B. auch entsprechende Formulierungen verstanden.

Bei den dermatologischen, kosmetischen und pharmazeutischen Produkten kann es sich beispielsweise um wässrige, wässrig-alkoholische, wässrig-tensidische oder alkoholische Mittel oder um Mittel auf Ölbasis, inklusive Mittel auf Ölbasis in wasserfreier Form oder um Emulsionen, Suspensionen oder Dispersionen handeln und zwar in Form von Fluids, Schäumen, Sprays, Gelen, Mousse, Lotionen, Cremes, Pudern oder Feuchttüchern (Wet Wipes).

In einer bevorzugten Ausführungsform werden die erfindungsgemäßen Mittel zum Konservieren von Feuchttüchern verwendet. Hierbei kann es sich bei der auf das textile Gewebe aufgetragenen, zu konservierenden Formulierung um eine Emulsion, insbesondere eine O/W Emulsion, aber auch um eine tensidische Formulierung oder ein öliges Mittel handeln.

In einer weiteren bevorzugten Ausführungsform werden die erfindungsgemäßen Mittel zum Konservieren von Emulsionen verwendet.

Bei den Emulsionen kann es sich sowohl um Wasser-in-Öl-Emulsionen als auch Öl-in-Wasser-Emulsionen, Mikroemulsionen, Nanoemulsionen und multiple Emulsionen handeln. Die Herstellung der Emulsionen kann in bekannter Weise, d.h. beispielsweise durch Kalt-, Heiß-, Heiß/Kalt- oder PIT-Emulgierung erfolgen. Eine besonders bevorzugte Ausführungsform sind selbstschäumende, schaumförmige, nachschäumende oder schäumbare Emulsionen und Mikroemulsionen.

Weiterer Gegenstand der vorliegenden Erfindung sind kosmetische, dermatologische oder pharmazeutische Formulierungen, die unter Verwendung eines Konservierungsmittels hergestellt worden sind, das
a) ein oder mehrere 1-Hydroxy-4-methyl-6-alkyl-2(1H)-pyridone und/oder ein oder mehrere Salze davon wobei alkyl eine lineare, verzweigte oder cyclische Alkylgruppe mit 1 bis 12 Kohlenstoffatomen, vorzugsweise 2,4,4-Trimethylpentyl oder Cyclohexyl und besonders bevorzugt 2,4,4-Trimethylpentyl, bedeutet,
b) einen oder mehrere Alkohole, enthaltend ein oder mehrere aromatische Gruppen und
c) ein oder mehrere weitere antimikrobielle Wirkstoffe
enthält bzw. kosmetische, dermatologische oder pharmazeutische Formulierungen, die ein derartiges Konservierungsmittel enthalten.

Weiterer Gegenstand der vorliegenden Erfindung sind kosmetische, dermatologische oder pharmazeutische Formulierungen enthaltend
a) ein oder mehrere 1-Hydroxy-4-methyl-6-alkyl-2(1H)-pyridone und/oder ein oder mehrere Salze davon wobei Alkyl eine lineare, verzweigte oder cyclische Alkylgruppe mit 1 bis 12 Kohlenstoffatomen, vorzugsweise 2,4,4-Trimethylpentyl oder Cyclohexyl und besonders bevorzugt 2,4,4-Trimethylpentyl, bedeutet,
b) einen oder mehrere Alkohole, enthaltend ein oder mehrere aromatische Gruppen und
c) ein oder mehrere weitere antimikrobielle Wirkstoffe.

Unter den genannten Formulierungen sind solche bevorzugt, in denen der eine oder die mehreren antimikrobiellen Wirkstoffe der Komponente c) ausgewählt sind aus Parabenen, vorzugsweise Methylparaben, Ethylparaben, Propylparaben, Butylparaben, Isobutylparaben, Natrium-Methylparaben, Natrium-Ethylparaben, Natrium-Propylparaben, Natriumisobutylparaben und/oder Natrium-Butylparaben, organischen Säuren, vorzugsweise Sorbinsäure, Kaliumsorbat, Salicylsäure, Natriumsalicylat, Benzoesäure und/oder Natriumbenzoat, Formaldehydabspaltern, vorzugsweise Imidazolidinyl Harnstoff, Diazolidinyl Harnstoff, DMDM Hydantoin und/oder Natrium Hydroxymethylglycinat und halogenierten Konservierungsstoffen, vorzugsweise lodopropynyl Butylcarbamat und/oder 2-Bromo-2-Nitropropan-1,3-diol.

Besonders bevorzugt sind kosmetische, dermatologische oder pharmazeutische Formulierungen enthaltend
a) Aminoethanolsalz von 1-Hydroxy-4-methyl-6-(2,4,4-trimethylpentyl)-2(1H)-pyridon,
b) 2-Phenoxyethanol und
c) eine oder mehrere Substanzen ausgewählt aus Methylparaben, Benzoesäure, Natriumbenzoat, Sorbinsäure, Kaliumsorbat, Salicylsäure und Natriumsalicylat.

In einer insbesondere bevorzugten Ausführungsform der Erfindung sind die Substanzen der Komponenten a), b) und c), bezogen auf die fertigen Formulierungen, zu 0,1 bis 2 Gew.-% in den Formulierungen enthalten.

In einer weiteren bevorzugten Ausführungsform der Erfindung handelt es sich bei den dermatologischen, kosmetischen und pharmazeutischen Produkten um Rinse-off Produkte, insbesondere um Shampoos, Haarspülungen, Haarkuren, Duschbäder, Duschgels oder Schaumbäder.

In einer weiteren bevorzugten Ausführungsform der Erfindung handelt es sich bei den dermatologischen, kosmetischen und pharmazeutischen Produkten um Leave-on Produkte, insbesondere um Tagescremes, Nachtcremes, Pflegecremes, Nährcremes, Bodylotions, Salben oder Lippenpflegemittel.

Weitere bevorzugte Leave-on Produkte sind dekorative Kosmetika, insbesondere Make-ups, eye-shadows, Lippenstifte oder Mascara.

In einer weiteren bevorzugten Ausführungsform der Erfindung handelt es sich bei den dermatologischen, kosmetischen und pharmazeutischen Produkten um Sonnenschutzmittel. Diese enthalten einen oder mehrere UV-Filter auf organischer oder anorganischer Basis.

In einer weiteren bevorzugten Ausführungsform der Erfindung handelt es sich bei den dermatologischen, kosmetischen und pharmazeutischen Produkten um Deodorantien und Antiperspirantien, insbesondere in Form von Sprays, Sticks, Gelen oder Lotionen.

In einer weiteren bevorzugten Ausführungsform der Erfindung handelt es sich bei den dermatologischen, kosmetischen und pharmazeutischen Produkten um tensidfreie Mittel, insbesondere um tensidfreie feste Mittel oder um tensidfreie Emulsionen.

Die dermatologischen, kosmetischen und pharmazeutischen Produkte können als weitere Hilfs- und Zusatzstoffe Tenside, Emulgatoren, kationische Polymere, Verdicker, Filmbildner, antimikrobielle Wirkstoffe, Adstringentien, Antioxidation, UV-Lichtschutzfilter, Pigmente/Mikropigmente, Gelierungsmittel, sowie weitere in der Kosmetik gebräuchliche Zusätze, wie z.B. Überfettungsmittel, feuchtigkeitsspendende Mittel, Silicone, Stabilisatoren, Konditioniermittel, Glycerin, Konservierungsmittel, Perlglanzmittel, Farbstoffe, Duft- und Parfümöle, Lösungsmittel, Hydrotrope, Trübungsmittel, Fettalkohole, Stoffe mit keratolytischer und keratoplastischer Wirkung, Antischuppenmittel, biogene Wirkstoffe (Lokalanästhetika, Antibiotika, Antiphlogistik, Antiallergica, Corticosteroide, Sebostatika), Vitamine, Bisabolol^{®}, Allantoin^{®}, Phytantriol^{®}, Panthenol^{®}, AHA-Säuren, Pflanzenextrakte, beispielsweise Aloe Vera und Proteine enthalten.

1-Hydroxy-4-methyl-6-(2,4,4-trimethylpentyl)-2(1 H)-pyridon und dessen Salze aus anorganischen und organischen Säuren werden nach den in EP 241 918, EP 646 368, DE 17 95 270, DE 22 14 608 und DE 22 34 009 beschriebenen Methoden erhalten.

Die Herstellung der erfindungsgemäßen Konservierungsmittel kann beispielsweise durch Zusammengeben der einzelnen Komponenten, gegebenenfalls unter leichtem Erwärmen auf ca. 35°C, erfolgen.

Die nachfolgenden Beispiele und Anwendungen sollen die Erfindung näher erläutern, ohne sie jedoch darauf zu beschränken (bei allen Prozentangaben handelt es sich um Gewichtsprozent).

### Beispiele:

### Beispiel 1: Konservierungsmittel 1

| | |
|---|---|
| Octopirox^{®} | 2 Gew.-% |
| Wasser | 1 Gew.-% |
| 2-Phenoxyethanol | 97 Gew.-% |

### Beispiel 2: Konservierungsmittel 2

| | |
|---|---|
| Octopirox^{®} | 5 Gew.-% |
| Wasser | 2,5 Gew.-% |
| 2-Phenoxyethanol | 92,5 Gew.-% |

### Beispiel 3: Konservierungsmittel 3

| | |
|---|---|
| Octopirox^{®} | 5 Gew.-% |
| Wasser | 1 Gew.-% |
| 2-Phenoxyethanol | 94 Gew.-% |

### Beispiel 4: Konservierungsmittel 4

| | |
|---|---|
| Octopirox^{®} | 10Gew.-% |
| Wasser | 5 Gew.-% |
| 2-Phenoxyethanol | 85 Gew.-% |

### Beispiel 5: Konservierungsmittel 5

| | |
|---|---|
| Octopirox^{®} | 10 Gew.-% |
| Wasser | 2 Gew.-% |
| 2-Phenoxyethanol | 88 Gew.-% |

### Herstellung Beispiele 1 bis 5:

Octopirox^{®} wird unter leichtem Erwärmen (20 bis 40°C) unter Rühren in 2-Phenoxyethanol gelöst. Anschließend wird Wasser unter Rühren zugegeben.

### Beispiel 6: Konservierungsmittel 6

| | |
|---|---|
| Octopirox^{®} | 5 Gew.-% |
| Nipagin^{®} M | 15 Gew.-% |
| Wasser | 2,5 Gew.-% |
| 2-Phenoxyethanol | 77,5 Gew.-% |

### Beispiel 7: Konservierungsmittel 7

| | |
|---|---|
| Octopirox^{®} | 5 Gew.-% |
| Natriumbenzoat | 15 Gew.-% |
| Wasser | 29 Gew.-% |
| 2-Phenoxyethanol | 51 Gew.-% |

### Beispiel 8: Konservierungsmittel 8

| | |
|---|---|
| Octopirox^{®} | 5 Gew.-% |
| Benzoesäure | 15 Gew.-% |
| Wasser | 2,5 Gew.-% |
| 2-Phenoxyethanol | 77,5 Gew.-% |

### Beispiel 9: Konservierungsmittel 9

| | |
|---|---|
| Octopirox^{®} | 5 Gew.-% |
| Kaliumsorbat | 15 Gew.-% |
| Wasser | 25 Gew.-% |
| 2-Phenoxyethanol | 55 Gew.-% |

### Beispiel 10: Konservierungsmittel 10

| | |
|---|---|
| Octopirox^{®} | 5 Gew.-% |
| Sorbinsäure | 10 Gew.-% |
| Propylenglykol | 45 Gew.-% |
| 2-Phenoxyethanol | 40 Gew.-% |

### Beispiel 11: Konservierungsmittel 11

| | |
|---|---|
| Octopirox^{®} | 5 Gew.-% |
| Natriumsalicylat | 15 Gew.-% |
| Wasser | 21 Gew.-% |
| 2-Phenoxyethanol | 59 Gew.-% |

### Beispiel 12: Konservierungsmittel 12

| | |
|---|---|
| Octopirox^{®} | 5 Gew.-% |
| Salicylsäure | 15 Gew.-% |
| Wasser | 2,5 Gew.-% |
| 2-Phenoxyethanol | 77,5 Gew.-% |

### Herstellung der Beispiele 6, 8, 10 und 12:

Octopirox^{®} und Nipagin^{®} M bzw. die organische Säure wird unter leichtem Erwärmen (20 bis 40°C) unter Rühren in 2-Phenoxyethanol bzw. einer 2-Phenoxyethanol/Propylenglykol Mischung gelöst. Anschließend wird gegebenenfalls Wasser unter Rühren zugegeben.

Herstellung der Beispiele 7, 9 und 11:
I Das Salz der organischen Säure wird unter leichtem Erwärmen (20 bis 40°C) in Wasser gelöst.
II Octopirox^{®} wird unter leichtem Erwärmen (20 bis 40°C) unter Rühren in 2-Phenoxyethanol gelöst.
III I wird unter Rühren zu II gegeben.

Bei den Konservierungsmitteln 1 bis 12 handelt es sich um klare Flüssigkeiten.

### Lagerstabilität:

Die wasserhaltigen Konservierungsmittel sind bei 0°C, 25°C und 40°C im Zeitraum von 3 Monaten stabil und zeigen keine Kristallisationstendenz.

**Tabelle 1: Minimale Hemmkonzentrationen (MHK) der Konservierungsmittel KM I und KM II im Vergleich zu 2-Phenoxyethanol (PE) und Octopirox^{®}. Die Zusammensetzungen von KM I und KM II sind in Tabelle 2 aufgeführt.**

| | KM I | % Octopirox^{®} berechnet KM I | % PE berechnet KM I | KM II | % Octopirox^{®} berechnet KM II | % PE berechnet KM II | Octopirox^{®} | PE |
|---|---|---|---|---|---|---|---|---|
| | MHK in % | | | MHK in % | | | MHK in % | MHK in % |
| Pseudomonas aeruginosa | 0.125 | 0.0025 | 0.1225 | 0.031 | 0.0031 | 0.0279 | 0.016 | 0.5 |
| Staphylococcus aureus | 0.125 | 0.0025 | 0.1225 | 0.031 | 0.0031 | 0.0279 | 0.002 | 0.25 |
| Aspergillus niger | 0.125 | 0.0025 | 0.1225 | 0.016 | 0.0016 | 0.0144 | 0.004 | 0.25 |
| Candida albicans | 0.063 | 0.00126 | 0.06174 | 0.016 | 0.0016 | 0.0144 | 0.004 | 0.5 |

Die MHK-Werte wurden nach DIN 58940 nach dem Agardilutionsverfahren ermittelt.
Die MHK-Werte zeigen, dass mit den Konservierungsmitteln KM I und KM II, bestehend aus den in Tabelle 2 genannten Komponenten Octopirox^{®} und 2-Phenoxyethanol im Vergleich zu den Einzelsubstanzen bei wesentlich geringeren Einsatzkonzentrationen eine ausreichende Konservierung erreicht werden kann.

**Tabelle 2: Konservierungsmittel KM I und KM II**

| | KM I | KM II |
|---|---|---|
| Octopirox^{®} [Gew.-%] | 2 | 10 |
| Phenoxyethanol [Gew.-%] | 98 | 90 |

Im Folgenden wurde die Wirksamkeit eines Konservierungsmittels enthaltend zwei Aktivkomponenten und verschiedener Konservierungsmittel enthaltend 3 Aktivkomponenten gegenüber verschiedenen Testorganismen bestimmt. Die Ergebnisse sind in Tabelle 3 dargestellt.

**Tabelle 3: Wirksamkeit der Konservierungsmittel KM III bis KM VII**

| Testorganismen | KM III | KM IV | KM V | KM VI | KM VII |
|---|---|---|---|---|---|
| Pseudomonas aeruginosa | A | A | A | A | F |
| Staphylococcus aureus | A | A | A | A | A |
| Candida albicans | A | A | A | A | A |
| Aspergillus niger | A | A | A | A | B |

Die in der Tabelle 3 aufgeführten Bewertungen "A" und "B" erfolgten analog der Europäischen Pharmacopoe 5.0, Abschitt 5.1.3 Efficacy of antimicrobial preservation. "F" bedeutet, dass die Bewertung "A" oder "B" nicht erreicht worden ist.

Die Zusammensetzung der Konservierungsmittel KM III bis KM VII sind in Tabelle 4 aufgeführt.

**Tabelle 4: Konservierungsmittel KM III bis KM VII**

| | KM III | KM IV | KM V | KM VI | KM VII |
|---|---|---|---|---|---|
| Octopirox^{®} [Gew.-%] | 5 | 5 | 5 | 5 | 5 |
| Nipagin^{®} M [Gew.-%] | 15 | - | - | - | - |
| Benzoesäure [Gew.-%] | - | 15 | - | - | - |
| Sorbinsäure [Gew.-%] | - | - | 10 | - | - |
| Salicylsäure [Gew.-%] | - | - | - | 15 | - |
| Propylene Glykol [Gew.-%] | - | - | 45 | - | - |
| Wasser [Gew.-%] | 2,5 | 2,5 | - | 2,5 | 2,5 |
| 2- Phenoxyethanol [Gew.-%] | 77,5 | 77,5 | 40 | 77,5 | 92,5 |

Die Wirksamkeit der Konservierungsmittel KM III bis KM VI gegenüber KM VII wurde in einer Tensidformulierung mit pH 7 mittels eines Konservierungsmittelbelastungstests überprüft. Die Konservierungsmittel KM III bis KM VII waren zu jeweils 0,5 Gew.-% in der Tensidformulierung enthalten. Der Konservierungsmittelbelastungstest wurde nach der Europäischen Pharmacopoe 5.0, Abschitt 5.1.3 Efficacy of antimicrobial preservation, durchgeführt und bewertet.

Die für die Konservierungsmittelbelastungstests verwendete Tensidformulierung hat folgende Zusammensetzung:

| | | | Gew.-% |
|---|---|---|---|
| A | GENAPOL^{®} LRO Paste | (Clariant) | 13,70 |
| B | Wasser | | ad 100 |
| C. | GENAGEN^{®} KB | (Clariant) | 6,00 |
| | Natriumchlorid | | 1,40 |
| D | Zitronensäure, Natriumhydroxid | (10 % in Wasser) | 0,08 |

### Herstellung

I A und B mischen.
II Zugabe von C zu I.
III Einstellen des pH-Werts mit D auf ca. 7

Die Ergebnisse (s. Tabelle 3) zeigen, dass mit den Konservierungsmitteln KM III bis KM VI, bestehend aus den in Tabelle 4 genannten Komponenten Octopirox^{®} und 2-Phenoxyethanol sowie
- Nipagin^{®} M und Wasser,
- Benzoesäure und Wasser,
- Sorbinsäure und Propylenglykol oder
- Salicylsäure und Wasser
im Vergleich zu dem Konservierungsmittel KM VII, bestehend aus den in Tabelle 4 genannten Komponenten Octopirox^{®}, 2-Phenoxyethanol und Wasser, eine signifikant bessere Konservierungsleistung erzielt werden kann.

### Formulierungsbeispiele

### Beispiel A

### Emulgatorfreie Feuchtigkeitscreme

| | | | Gew.-% |
|---|---|---|---|
| A | Mineralöl, niedrigviskos | | 6,00 |
| | Isopropylpalmitat | | 3,60 |
| | Sojaöl | | 2,40 |
| B | Aristoflex^{®} AVC | (Clariant) | 1,00 |
| C | Wasser | | ad 100 |
| | Glycerin | | 4,00 |
| | Konservierungsmittel aus Beispiel 2 | (Clariant) | 0,50 |
| D | Duftstoff | | 0,30 |

### Herstellung

I Mischen von A und B bei Raumtemperatur
II Einrühren von C in I, dann Zugabe von D
III Homogenisieren der Emulsion

### Beispiel B

### O/W-Nachtcreme

| | | | Gew.-% |
|---|---|---|---|
| A | Hostaphat^{®} KW 340 D | (Clariant) | 2,00 |
| | Hostacerin^{®} DGMS | (Clariant) | 3,00 |
| | Stearylsäure | | 2,00 |
| | Cetylalkohol | | 2,00 |
| | Cetylpalmitat | | 1,00 |
| | Lunacera^{®} M | | 1,00 |
| | Miglyol^{®} 812 | | 7,00 |
| | Abil^{®} 100 | | 1,00 |
| | Mineralöl, niedrigviskos | | 5,00 |
| | Jojobaöl | | 2,00 |
| | Tocopherolacetat | | 1,00 |
| B | Carbopol^{®} 980 | | 0,20 |
| C | Natriumhydroxid (10 %ig in Wasser) | | 0,80 |
| | Glycerin | | 3,00 |
| | Wasser | | ad 100 |
| | Konservierungsmittel aus Beispiel 4 | (Clariant) | 0,80 |
| D | Duftstoff | | 0,40 |

### Herstellung

I Schmelzen von A bei ca. 70°C, dann Zugabe von B
II Erwärmen von C auf ca. 70°C
III Einrühren von II in I und Rühren bis zum Abkühlen auf Raumtemperatur
IV Zugabe von D zu III bei ca. 35°C
V Homogenisieren der Lösung

### Beispiel C

### Emulsion für Feuchttücher

| | | | Gew.-% |
|---|---|---|---|
| A | EMULSOGEN^{®} HCP 049 | (Clariant) | 0,50 |
| | HOSTAPHAT^{®} KML | (Clariant) | 1,00 |
| | Myritol^{®} 318 | | 0,50 |
| | Mineralöl, niedrig viskos | | 0,50 |
| B | Tocopheryl Acetat | | 0,20 |
| | Panthenol | | 0,50 |
| C | Wasser | | ad 100 |
| | HOSTAPON^{®} CLG | (Clariant) | 0,20 |
| | Zitronensäure (10 %ig in Wasser) | | 0,90 |
| | EDTA, Na-Salz | | 0,05 |
| | NaOH (10 %ig in Wasser) | | 0,45 |
| | ALLANTOIN | (Clariant) | 0,20 |
| | Konservierungsmittel aus Beispiel 3 | (Clariant) | 1,00 |

### Herstellung

I B zu A geben
II C auf ca. 40°C erhitzen.
III II unter Rühren zu I geben und kaltrühren.

### Beispiel D

### Cremespülung zur Haarspflege

| | | | Gew.-% |
|---|---|---|---|
| A | GENAMIN^{®} KDMP | (Clariant) | 2,00 |
| | HOSTAPHAT^{®} KL 340 D | (Clariant) | 1,50 |
| | Cetyl Stearyl Alkohol | | 2,00 |
| | Paraffinöl, hoch- viskos | | 2,00 |
| B | Wasser | | ad 100,00 |
| | Konservierungsmittel aus Beispiel 1 | (Clariant) | 0,50 |
| C | Duftstoff | | 0,30 |
| | Farbstoff | | q.s. |
| D | Zitronensäure | | . q.s. |

### Herstellung

I A bei 75°C aufschmelzen.
II B auf 75°C erhitzen.
III II zu I geben unter Rühren.
IV Kaltrühren.
V Bei 35°C die Komponenten von C zu IV geben.
VI Abschließend den pH- Wert mit D auf 4 einstellen.

### Beispiel E

### Shampoo für coloriertes Haar

| | | | Gew.-% |
|---|---|---|---|
| A | UCARE^{®} Polymer JR-400 | | 0,30 |
| B | Wasser | | ad 100 |
| C | GENAPOL^{®} LRO liquid | (Clariant) | 42,90 |
| | GENAGEN^{®} LAA | (Clariant) | 6,70 |
| | Duftstoff | | 0,40 |
| | Farbstofflösung | | q.s. |
| | Konservierungsmittel aus Beispiel 1 | (Clariant) | 0,75 |
| | GENAGEN^{®} CAB 818 | (Clariant) | 6,70 |
| | Extrapone Henna Spezial | | 2,00 |
| | Extrapone Eibisch Spezial | | 1,00 |
| | Uvinul MS 40 | | 0,50 |
| D | Zitronensäure (25 %ig in Wasser) | | q.s. |
| E | Natriumchlorid | | 1,00 |

### Herstellung

I A bei 50°C in B lösen.
II Nacheinander die Komponenten von C zu I geben.
III Einstellen des pH- Wertes mit D auf 5,5.
IV Abschließend die Viskosität mit E einstellen.

### Beispiel F

### Conditioner- Shampoo für splissanfälliges Haar

| | | | Gew.-% |
|---|---|---|---|
| A | GENAMIN^{®} BTLF | (Clariant) | 2,00 |
| | GENAPOL^{®} PMS | (Clariant) | 1,50 |
| | GENAPOL^{®} LRO liquid | (Clariant) | 35,00 |
| | Glucamate DOE 120 | | 2,50 |
| | Cetyl Alkohol | | 0,50 |
| | Natriumchlorid | | 0,50 |
| | Natriumcumensulfonat | | 0,50 |
| B | Wasser | | ad 100 |
| C | GENAGEN^{®} CAB 818 | (Clariant) | 6,00 |
| | HOSTAPON^{®} KCG | (Clariant) | 8,00 |
| D | Farbstofflösung | | q.s. |
| | Konservierungsmittel aus Beispiel 6 | (Clariant) | 0,60 |
| | Duftstoff | | 0,30 |
| | D- Panthenol | | 0,50 |
| | SilCare^{®} SILICONE 41 M15 | (Clariant) | 0,50 |
| E | Natriumchlorid | | 0,50 |

### Herstellung

I Lösen und Aufschmelzen der Komponenten von A in B unter Rühren und Erhitzen auf ca. 85°C.
II Kaltrühren.
III Die Komponenten von C zu II geben.
IV Falls nötig den pH-Wert einstellen.
V Die Komponenten von D zu IV geben.
VI Abschließend die Viskosität mit E einstellen.

### Beispiel G

### OW- Sonnenmilch

| | | | Gew.-% |
|---|---|---|---|
| A | HOSTAPHAT^{®} KL 340 D | (Clariant) | 3,00 |
| | HOSTACERIN^{®} DGSB | (Clariant) | 5,00 |
| | Paraffinöl, niedrig- viskos | | 6,00 |
| | Isopropyl Palmitate | | 6,00 |
| | Cetiol^{®} V | | 6,00 |
| | Neo Heliopan^{®} AV | | 4,00 |
| | Avocado Oil | | 1,00 |
| | Panthenol | | 0,50 |
| B | Carbopol^{®} 980 | | 0,40 |
| C | Natronlauge (10 % in Wasser) | | 1,60 |
| | Glycerin | | 3,00 |
| | Wasser | | ad 100 |
| | Konservierungsmittel aus Beispiel 7 | (Clariant) | 1,00 |
| D | Duftstoff | | 0,30 |

### Herstellung

I A bei ca. 70°C aufschmelzen, danach B zugeben.
II C auf ca. 70°C erwärmen.
III II in I rühren und kaltrühren.
IV. Bei ca. 35°C D zu III geben.
V Abschließend die Emulsion homogenisieren.

### Beispiel H

### WO Sonnenmilch

| | | | Gew.-% |
|---|---|---|---|
| A | HOSTACERIN° WO | (Clariant) | 2,00 |
| | Arlacel 989 | | 2,00 |
| | Paraffinöl, niedrig- viskos | | 10,00 |
| | Eutanol G | | 5,00 |
| | Isopropyl Palmitate | | 5,00. |
| | Neo Heliopan^{®} E 1000 | | 4,00 |
| | Neo Heliopan^{®} BB | | 1,00 |
| B | Natriumchlorid | | 2,00 |
| | Wasser | | ad 100 |
| | Konservierungsmittel aus Beispiel 5 | (Clariant) | 0,80 |
| C | Duftstoff | | 0,30 |

### Herstellung

I A bei ca. 80°C aufschmelzen.
II Die kalte Lösung von B in I rühren.
III Kaltrühren.
IV C bei ca. 35°C zu III geben.

### Beispiel I

### WO Creme

| | | | Gew.-% |
|---|---|---|---|
| A | HOSTACERIN^{®} WO | (Clariant) | 10,00 |
| | Permulgin^{®} 3510 | | 4,00 |
| | Paraffinöl, niedrig- viskos | | 7,00 |
| | Isopropyl Palmitat | | 5,00 |
| | Sonnenblumenöl | | 5,00 |
| | Mandelöl | | 3,00 |
| | Weizenkeimöl | | 2,00 |
| | Antioxidant | | q.s. |
| B | Wasser | | ad 100 |
| | Glycerin | | 4,00 |
| | Magnesium Sulfat | | 1,00 |
| | Zitronensäure (10 % in Wasser) | | 0,25 |
| | Tetranatrium EDTA | | 0,10 |
| C | Duftstoff | | 0,40 |
| | Konservierungsmittel aus Beispiel 8 | (Clariant) | 0,80 |

### Herstellung

I A bei ca. 80°C aufschmelzen.
II B auf ca. 80°C erwärmen.
III II in I rühren und kaltrühren.
IV C bei ca. 35°C zu III geben.

### Beispiel J

### OW Hautmilch

| | | | Gew.-% |
|---|---|---|---|
| A | Hostaphat^{®} KL 340 D | (Clariant) | 1,00 |
| | Paraffinöl, niedrig- viskos | | 8,00 |
| | Isopropyl Palmitat | | 3,00 |
| | Cetearyl Alkohol | | 0,50 |
| | Myritol^{®} 318 | | 2,00 |
| | Tegin^{®} M | | 0,50 |
| | Tocopheryl Acetate | | 1,00 |
| | SilCare^{®} Silicone 41M15 | (Clariant) | 1,00 |
| B | Aristoflex^{®} HMB | | 0,50 |
| C | Wasser | | ad 100 |
| | Glycerin | | 5,00 |
| D | Duftstoff | | 0,30 |
| | Alcohol | | 1,00 |
| | Konservierungsmittel aus Beispiel 9 | (Clariant) | 0,75 |

### Herstellung

I A bei ca. 60°C aufschmelzen, danach B zugeben.
II C auf ca. 60°C erwärmen.
III II in I rühren, und kaltrühren.
IV Die Komponenten von D werden nacheinander bei ca. 35°C zu III gegeben.
V Abschließend die Emulsion homogenisieren.

### Beispiel K

### Haar- Stylingcreme

| | | | Gew.-% |
|---|---|---|---|
| A | Wasser | | ad 100 |
| | Polyglykol 3000 S | (Clariant) | 1,00 |
| | Propylen Glykol | | 1,00 |
| | Serenoa Serrulata | | 0,30 |
| | Natronlauge (50 %ig in Wasser) | | 0,30 |
| B | Cera Alba | | 8,00 |
| | PEG-20 Stearate | | 5,00 |
| | Cetearyl Alkohol | | 5,00 |
| | Isopropylmyristat | | 5,00 |
| | Ozokerite Wax | | 5,00 |
| | Genamin^{®} KDMP | (Clariant) | 1,50 |
| | Hostaphat^{®} KL 340 D | (Clariant) | 2,00 |
| C | Diaformer^{®} Z-651 | (Clariant) | 1,00 |
| | Duftstoff | | 0,30 |
| | Konservierungsmittel aus Beispiel 11 | (Clariant) | 0,90 |

### Herstellung

I A auf ca. 80°C erhitzen.
II B auf ca. 80°C erhitzen.
III I in II rühren und kaltrühren.
IV Bei ca. 35°C die Komponenten von C zu III geben.

### Beispiel L

### Reinigendes und pflegendes Gesichtswasser

| | | | Gew.-% |
|---|---|---|---|
| A | Glycerin | | 10,00 |
| | Polyglykol 400 | (Clariant) | 5,00 |
| | Panthenol | | 0,50 |
| | Duftstoff | | 0,20 |
| | Konservierungsmittel aus Beispiel 10 | (Clariant) | 0,80 |
| | ALLANTOIN (Clariant) | | 0,10 |
| | Niacinamide | | 0,10 |
| | Extrapon Hamamelis | | 1,00 |
| B | Wasser | | ad 100 |
| C | Aristoflex ^{®} HMB | (Clariant) | 0,30 |

### Herstellung

I A in B unter Rühren lösen.
II C unter Rühren zu I geben, rühren bis homogen.

### Beispiel M

### Körperwaschlotion

| | | | Gew.-% |
|---|---|---|---|
| A | HOSTAPON^{®} CCG | (Clariant) | 30,00 |
| | Wasser | | ad 100 |
| B | Glucamate^{®} DOE 120 | | 2,50 |
| C | GENAGEN^{®} CAB | (Clariant) | 10,00 |
| | GENAPOL^{®} PDB | (Clariant) | 3,00 |
| | Duftstoff | | 0,30 |
| | Farbstofflösung | | q.s. |
| | Konservierungsmittel aus Beispiel 12 | (Clariant) | 0,50 |
| D | Zitronensäure (50 % in Wasser) | | 3,30 |
| E | Natriumchlorid | | 0,60 |

### Herstellung

I B unter Rühren in A lösen unter leichtem Erwärmen.
II Nacheinander die Komponenten von C zu I geben.
III Einstellen des pH-Wertes mit D.
IV Abschließend die Viskosität mit E einstellen.

### Beispiel N

### Duschgel

| | | | Gew.-% |
|---|---|---|---|
| A | GENAPOL^{®} LRO liquid | (Clariant) | 30,00 |
| B | HOSTAPON^{®} KCG | (Clariant) | 5,00 |
| | Duftstoff | | 0,50 |
| | Cetiol^{®} HE | | 2,00 |
| C | ALLANTOIN | (Clariant) | 0,40 |
| D | Wasser | | 52,60 |
| E | GENAGEN^{®} CAB | (Clariant) | 6,00 |
| | GENAPOL^{®} L-3 | (Clariant) | 2,00 |
| | Farbstofflösung | | q.s. |
| | Konservierungsmittel aus Beispiel 6 | (Clariant) | 0,70 |
| F | Natriumchlorid | | 1,50 |

### Herstellung

I Die Komponenten von B nacheinander zu A geben.
II C in leicht warmen D lösen.
III II in I rühren.
IV Nacheinander die Komponenten von E zu III geben.
V Falls notwendig den pH-Wert einstellen.
VI Abschließend die Viskosität mit F einstellen.

### Beispiel O

### Cremespülung zur Haarspflege

| | | | Gew.-% |
|---|---|---|---|
| A | Genamin^{®} KDMP | (Clariant) | 1,00 |
| | Genamin^{®} CTAC | (Clariant) | 2,00 |
| | Hostaphat^{®} KML | (Clariant) | 1,50 |
| | Cetyl Alkohol | | 2,50 |
| B | Wasser | | ad 100 |
| | entspricht Konservierungsmittel aus Beispiel 12 | (Clariant) | 0,40 |
| C | Duftstoff | | 0,30 |
| | Farbstofflösung | | q.s. |
| | Dow Corning 949 | | 1,00 |

### Herstellung

I A bei ca. 75°C aufschmelzen.
II B auf ca. 75°C erhitzen.
III II unter Rühren zu I geben und anschließend kaltrühren.
IV Bei ca. 30°C die Komponenten von C zu III geben.
V Abschließend den pH-Wert auf 4,0 einstellen.

### Beispiel P

### Haarspitzenfluid

| | | | Gew.-% |
|---|---|---|---|
| A | SilCare Silicone^{®} 41 M15 | (Clariant) | 0,30 |
| B | GENAPOL^{®} LA 070 | (Clariant) | 8,00 |
| C | Glucamate DOE 120 | | 4,00 |
| D | Wasser | | ad 100 |
| E | Biobranil | | 0,50 |
| | Glycerin | | 2,00 |
| | Panthenol | | 0,50 |
| F | SilCare^{®} Silicone SEA | (Clariant) | 0,50 |
| | GENAMIN^{®} CTAC | (Clariant) | 2,00 |
| | entspricht Konservierungsmittel aus Beispiel 7 | (Clariant) | 0,80 |

### Herstellung

I Solubilisiere A in B.
II C in warmen D lösen.
III II zu I geben.
IV Die Komponenten von E zu III geben.
V Bei ca. 35°C die Komponenten von F unter Rühren zu IV geben.

### Beispiel Q

### Haargel

| | | | Gew.-% |
|---|---|---|---|
| A | GENAPOL^{®} HS 200 | (Clariant) | 0,20 |
| | Duftstoff | | 0,20 |
| B | Wasser | | ad 100 |
| | GENAPOL^{®} HS 200 | (Clariant) | 1,80 |
| C | Propylene Glycol | | 2,00 |
| | Diaformer^{®} Z-651 | (Clariant) | 4,50 |
| | Alcohol | | 20,00 |
| D | Farbstofflösung | | q.s. |
| | Konservierungsmittel aus Beispiel 2 | (Clariant) | 0,30 |
| E | ARISTOFLEX^{®} AVC | (Clariant) | 0,80 |

### Herstellung

I Die Komponenten von A mischen.
II Die Komponenten von B unter leichtem Erwärmen lösen. Abkühlen lassen und zu I geben.
III Nacheinander die Komponenten von C zu 11 geben.
IV Die Komponenten von D zu III geben.
V abschließend E zu IV geben und rühren bi seine homogene Formulierung entsteht.

### Beispiel R

### Stylingspray für lockiges Haar

| | | | Gew.-% |
|---|---|---|---|
| A | Deionized Water | | ad 100 |
| | Dow Corning 190 Surfactant | | 0,30 |
| B | Diaformer^{®} Z - 632 | (Clariant) | 1,00 |
| | Aristoflex^{®} A 60 | (Clariant) | 1,50 |
| C | Isopropyl Alkohol | | 45,00 |
| | Duftstoff | | 0,30 |
| D | Niacinamid | | 0,30 |
| | Genamin^{®} CTAC | (Clariant) | 0,50 |
| | Panthenol | | 0,20 |
| | Uvinul MS 40 | | 0,20 |
| | Merquat 550 | | 0,30 |
| | Konservierungsmittel aus Beispiel 6 | (Clariant) | 0,30 |
| E | Zitronensäure | | qs. |

### Herstellung

I Die Komponenten von A gut mischen.
II Die Komponenten von B gut mischen und anschließend II zu I geben.
III Die Komponenten von C gut mischen und anschließend III zu II geben.
IV Nacheinander die Komponenten von D zu III geben.
V Abschließend den pH-Wert mit E auf 4,0 einstellen.

### INCI Bezeichnung der eingesetzten Handelsprodukte:

| | | |
|---|---|---|
| Abil^{®} 100 | | Dimethicone |
| ALLANTOIN | | Allantoin |
| Aristoflex^{®} AVC: | | Ammonium Acyloyldimethyltaurate/ VP Copolymer |
| Aristoflex^{®} A 60 | | VA Crotonates Copolymer (and) Isopropyl Alcohol |
| Aristoflex^{®} HMB | | Ammonium Acryloyldimethyltaurate/Beheneth-25 Methacrylate |
| Arlacel^{®} 989 | | Crosspolymer PEG-7 Hydrogenated Castor Oil |
| Biobranil | | Soybean (Glycine Soja) Oil (and) Wheat (Triticum Vulgare) Bran Lipids |
| Carbopol^{®} 980 | | Carbomer |
| Cetiol^{®} HE | | PEG-7 Glyceryl Cocoate |
| Cetiol V | | Decyloleate |
| Diaformer^{®} Z-632 | | Acrylates/Stearyl Acrylate/ Ethylamine Oxide Methacrylate Copolymer |
| Diaformer^{®} Z-651 | | Acrylates/Lauryl Acrylate/Stearyl Acrylate/Ethylamine Oxide Methacrylate Copolymer |
| Dow Coring^{®} 190 Surfactant | | PEG/PPG-18/18 Dimethicone |
| Dow Corning^{®} 949 | | Amodimethicone (and) Cetrimonium Chloride (and) Trideceth-12 |
| EDTA, Na-Salz | | Disodium EDTA |
| EMULSOGEN^{®} HCP 049 | | PEG-40 hydrogenated Castor Oil, (and) propylene glycol |
| Eutanol^{®} G | | Octyldodecanol |
| Extrapone Eibisch spezial | | Aqua (and) Ethoxydiglycol (and) Propylene Glycol (and) Althea Officinalis Extract (and) Butylene Glycol |
| Extrapone Henna spezial | | Aqua (and) Ethoxydiglycol (and) Propylene Glycol (and) Butylene Glycol (and) Henna (Lawsonia Inermis) Extract |
| Genagen^{®} CAB | | Cocamidopropyl Betaine |
| Genagen^{®} CAB 818 | | Cocamidopropyl Betaine |
| Genagen LAA | | Sodium Lauroamphoacetate |
| Genamin^{®} BTLF | | Behentrimonium Chloride |
| Genamin^{®} CTAC | | Cetrimonium Chloride |
| Genamin^{®} KDMP | | Behentrimonium Chloride |
| Genapol^{®} HS 200 | | Steareth-20 |
| Genapol^{®} LA 070 | | Laureth-7 |
| Genapol^{®} LRO liquid | | Sodium Laureth Sulfate |
| Genapol^{®} L-3 | | Laureth-3 |
| Genapol^{®} PDB | | Glycol Distearate (and) Laureth-4 (and) Cocamidopropyl Betaine |
| Genapol^{®} PMS | | Glycol Distearate. |
| Glucamate DOE 120 | | PEG-120 Methylglucose Dioleate |
| Hostacerin^{®} DGMS | (Clariant) | Polyglyceryl- 2 Stearate |
| Hostacerin DGSB | | PEG-4 Polyglyceryl-2 Stearate |
| Hostacerin^{®} WO | | Polyglyceryl-2 Sesquiisostearate (and) Beeswax (and) Microcrystalline Wax (and) Mineral Oil (and) Magnesium Stearate (and) Aluminum Stearate |
| Hostapha^{®} KL 340 D | | Trilaureth- 4 Phosphate |
| HOSTAPHAT^{®} KML | | Laureth-4 Phosphate (and) Polyglyceryl-2 Sesquiisostearate |
| Hostaphat^{®} KW 340 D | (Clariant) | Triceteareth-4 Phosphate |
| Hostapon^{®} CCG | | Sodium Cocoyl Glutamate |
| HOSTAPON^{®} CLG | | Sodium Lauroyl Glutamate |
| Hostapon^{®} KCG | | Sodium Cocoyl Glutamate |
| Lunacera^{®} M | | Microcrystalline Wax |
| Merquat 550 | | Polyquaternium-7 |
| Miglyol^{®} 812 | | Caprylic/Capric Triglyceride |
| Myritol^{®} 318 | | Caprylic-/Capriic Triglyceride |
| Neo Heliopan^{®} AV | | Ethylhexyl Methoxycinnamate |
| Neo Heliopan^{®} BB | | Benzophenone-3 |
| Neo Heliopan^{®} E 1000 | | Isoamyl p-Methoxycinnamate |
| Octopirox^{®} | Clariant GmbH | Piroctone Olamine (Chemische Bezeichnung: |
| 1-Hydroxy-4-methyl-6-(2,4,4-trimethylpentyl)-2(1H)-pyridon, 2-Aminoethanolsalz) | | |
| Permulgin^{®} 3510 | | Beeswax (and) Mineral Oil (and) Isopropylmyristate |
| Polyglykol 400 | | PEG-8 |
| Polyglykol 3000 S | | PEG-60 |
| SilCare^{®} Silicone SEA | | Trideceth-9 PG Amodimethicone (and) Trideceth-12 |
| SilCare^{®} Silicone 41M15 | | Caprylyl Trimethicone |
| Uvinul MS 40 | | Benzophenone- 4 |

## Patentansprüche

1. Klares, flüssiges Konservierungsmittel, enthaltend
a) 1-Hydroxy-4-methyl-6-(2,4,4-trimethylpentyl)-2(1H)-pyridon und/oder ein oder mehrere Salze davon in Gewichtsmengen von 0,1 bis 20 %, bevorzugt 1,1 bis 10 %, besonders bevorzugt 1,5 bis 7 % und insbesondere bevorzugt 5 % und
b) einen oder mehrere Alkohole ausgewählt aus 2-Phenoxyethanol, 1-Phenoxy 2-propanol und Benzylalkohol in Gewichtsmengen von 35 bis 99,9 % und vorzugsweise von 80 bis 99,9 %.

2. Konservierungsmittel gemäß Anspruch 1, **dadurch gekennzeichnet, dass** es
a) 1-Hydroxy-4-methyl-6-(2,4,4-trimethylpentyl)-2(1H)-pyridon und/oder ein oder mehrere Salze davon,
b) einen oder mehrere Alkohole, ausgewählt aus 2-Phenoxyethanol, 1-Phenoxy-2-propanol und Benzylalkohol,
c) Wasser,
d) optional einen oder mehrere weitere antimikrobielle Wirkstoffe,
e) optional ein oder mehrere Hydrotrope, und
f) optional ein oder mehrere weitere Additive
enthält

3. Konservierungsmittel gemäß Anspruch 1 oder 2, **dadurch gekennzeichnet, dass** es
a) 1-Hydroxy-4-methyl-6-(2,4,4-trimethylpentyl)-2(1H)-pyridon und/oder ein oder mehrere Salze davon in Gewichtsmengen von 0,1 bis 20 %, bevorzugt 1,1 bis 10 %, besonders bevorzugt 1,5 bis 7 % und insbesondere bevorzugt 5 %,
b) einen oder mehrere Alkohole, ausgewählt aus 2-Phenoxyethanol, 1-Phenoxy-2-propanol und Benzylalkohol in Gewichtsmengen von 35 bis 99,8 % und bevorzugt von 59,8 bis 99,8 % und
c) Wasser in Gewichtsmengen von 0,1 bis 35 %, bevorzugt 0,1 bis 20 %,
besonders bevorzugt 0,5 bis 5 % und insbesondere bevorzugt 1 bis 3 % enthält.

4. Konservierungsmittel, gemäß einem oder mehreren der Ansprüche 1 bis 3, **dadurch gekennzeichnet, dass** es das Aminoethanolsalz von 1-Hydroxy-4-methyl-6-(2,4,4-trimethylpentyl)-2(1H)-pyridon, enthält.

5. Konservierungsmittel gemäß einem oder mehreren der Ansprüche 1 bis 4, **dadurch gekennzeichnet, dass** der Alkohol der Komponente b) 2-Phenoxyethanol ist.

6. Konservierungsmittel gemäß einem oder mehreren der Ansprüche 1 bis 5, **dadurch gekennzeichnet, dass** es ein oder mehrere weitere antimikrobielle Wirkstoffe enthält.

7. Konservierungsmittel gemäß Anspruch 6, **dadurch gekennzeichnet, dass** es das eine oder die mehreren weiteren antimikrobiellen Wirkstoffe in einer Menge von 1 bis 25 Gew.-% enthält.

8. Konservierungsmittel nach Anspruch 6 oder 7, **dadurch gekennzeichnet, dass** das eine oder die mehreren weiteren antimikrobiellen Wirkstoffe ausgewählt sind aus Parabenen, vorzugsweise Methylparaben, Ethylparaben, Propylparaben, Butylparaben, Isobutylparaben, Natrium-Methylparaben, Natrium-Ethylparaben, Natrium-Propylparaben, Natriumisobutylparaben und/oder Natrium-Butylparaben, organischen Säuren, vorzugsweise Sorbinsäure, Kaliumsorbat, Salicylsäure, Natriumsalicylat, Benzoesäure und/oder Natriumbenzoat, Formaldehydabspaltern, vorzugsweise Imidazolidinyl Harnstoff, Diazolidinyl Harnstoff, DMDM Hydantoin und/oder Natrium Hydroxymethylglycinat und halogenierten Konservierungsstoffen, vorzugsweise Iodopropynyl Butylcarbamat und/oder 2-Bromo-2-Nitropropan-1,3-diol.

9. Konservierungsmittel nach Anspruch 8, **dadurch gekennzeichnet, dass** es
I) Aminoethanolsalz von 1-Hydroxy-4-methyl-6-(2,4,4-trimethytpentyl)-2(1H)-pyridon, 2-Phenoxyethanol und Methylparaben oder
II) Aminoethanolsalz von 1-Hydroxy-4-methyl-6-(2,4,4-trimethylpentyl)-2(1H)-pyridon, 2-Phenoxyethanol und Natriumbenzoat oder
III) Aminoethanolsalz von 1-Hydroxy-4-methyl-6-(2,4,4-trimethylpentyl)-2(1H)-pyridon, 2-Phenoxyethanol und Benzoesäure oder
IV) Aminoethanolsalz von 1-Hydroxy-4-methyl-6-(2,4,4-trimethylpentyl)-2(1H)-pyridon, 2-Phenoxyethanol und Kallumsorbat oder
V) Aminoethanolsalz von 1-Hydroxy-4-methyl-6-(2,4,4-trimethylpentyl)-2(1H)-pyridon, 2-Phenoxyethanol und Sorbinsäure oder
VI) Aminoethanolsalz von 1-Hydroxy-4-methyl-&(2,4,4-trimethylpentyl)-2(1H)-pyridon. 2-Phenoxyethanol und Natriumsalicylat oder
VII) Aminoethanolsalz von 1-Hydroxy-4-methyl-6-(2,4,4-trimethylpentyl)-2(1H)-pyridon, 2-Phenoxyethanol und Salicylsäure
enthält.

10. Konservierungsmittel gemäß einem oder mehreren der Ansprüche 1 bis 9, **dadurch gekennzeichnet, dass** es ein oder mehrere Antioxidantien, enthält.

11. Konservierungsmittel gemäß Anspruch 10, **dadurch gekennzeichnet, dass** das eine oder die mehreren Antioxidantien ausgewählt sind aus Tocopherylacetat, BHT und EDTA.

12. Konservierungsmittel gemäß einem oder mehreren der Ansprüche 1 bis 11, **dadurch gekennzeichnet, dass** es ein oder mehrere Hydrotrope enthält

13. Konservierungsmittel gemäß Anspruch 12, **dadurch gekennzeichnet, dass** das Hydrotrop Cumolsulfonat ist.

14. Konservierungsmittel gemäß einem oder mehreren der Ansprüche 1 bis 13, **dadurch gekennzeichnet, dass** es ein oder mehrere Solubilisatoren enthält.

15. Konservierungsmittel nach Anspruch 14, **dadurch gekennzeichnet, dass** das eine oder die mehreren Solubilisatoren ausgewählt sind aus Triethylenglykol, Butylenglykol und 1,2-Propylenglykol.

16. Konservierungsmittel gemäß einem oder mehreren der Ansprüche 1, 4 und 5, **dadurch gekennzeichnet, dass** es aus
a) 1-Hydroxy-4-methyl-6-(2,4,4-trimethylpentyl)-2(1 H)-pyridon und/oder ein oder mehreren Salzen davon und
b) einem oder mehreren Alkoholen ausgewählt aus 2-Phenoxyethanol, 1-Phenoxy-2-propanol und Benzylalkohol
besteht.

17. Konservierungsmittel gemäß einem oder mehreren der Ansprüche 1 bis 5, **dadurch gekennzeichnet, dass** es aus
a) 1-Hydroxy-4-methyl-6-(2,4,4-trimethylpentyl)-2(1H)-pyridon und/oder ein oder mehreren Salzen davon,
b) einem oder mehreren Alkoholen, ausgewählt aus 2-Phenoxyethanol, 1-Phenoxy-2-propanol und Benzylalkohol, und
c) Wasser
besteht.

18. Verwendung eines Mittels gemäß einem oder mehreren der Ansprüche 1 bis 17 zum Konservieren von dermatologischen, kosmetischen und pharmazeutischen Produkten.

19. Verwendung eines Mittels gemäß einem oder mehreren der Ansprüche 1 bis 17 zum Konservieren von Feuchttüchern.

20. Verwendung eines Mittels gemäß einem oder mehreren der Ansprüche 1 bis 17 zum Konservieren von Emulsionen.

21. Verwendung eines Mittels gemäß einem oder mehreren der Ansprüche 1 bis 17 zur Herstellung einer kosmetischen, dermatologischen oder pharmazeutischen Zubereitung.

22. Kosmetische, dermatologische oder pharmazeutische Formulierung enthaltend
a) Aminoethanolsalz von 1-Hydroxy-4-methyl-6-(2,4,4-trimethylpentyl)-2(1H)-pyridon,
b) 2-Phenoxyethanol und
c) eine oder mehrere Substanzen ausgewählt aus Benzoesäure, Natriumbenzoat, Sorbinsäure, Kaliumsorbat, Salicylsäure und Natriumsalicylat.

23. Formulierung nach Anspruch 22, **dadurch gekennzeichnet, dass** die Substanzen der Komponenten a), b) und c), bezogen auf die fertigen Formulierungen, zu 0,1 bis 2 Gew.% in den Formulierungen enthalten sind.

24. Formulierung nach Anspruch 22 oder 23, **dadurch gekennzeichnet, dass** es sich um eine Emulsion handelt.

25. Formulierung nach Anspruch 22 oder 23, **dadurch gekennzeichnet, dass** es sich um eine tensidfreie Emulsion handelt.

## Claims

1. A clear liquid preservative composition comprising
a) 1-hydroxy-4-methyl-6-(2,4,4-trimethylpentyl)-2(1H)-pyridone and/or one or more salts thereof in amounts by weight of 0.1% to 20%, preferably 1.1% to 10%, more preferably 1.5% to 7%, and with particular preference 5%, and
b) one or more alcohols selected from 2-phenoxyethanol, 1-phenoxy-2-propanol, and benzyl alcohol in amounts by weight of 35% to 99.9% and preferably of 80% to 99.9%.

2. The preservative composition of claim 1, **characterized in that** it comprises
a) 1-hydroxy-4-methyl-6-(2,4,4-trimethylpentyl)-2(1H)-pyridone and/or one or more salts thereof,
b) one or more alcohols selected from 2-phenoxyethanol, 1-phenoxy-2-propanol, and benzyl alcohol,
c) water,
d) optionally one or more further active antimicrobial substances,
e) optionally one or more hydrotropes, and
f) optionally one or more further additives.

3. The preservative composition of claim 1 or 2,
**characterized in that** it comprises
a) 1-hydroxy-4-methyl-6-(2,4,4-trimethylpentyl)-2(1H)-pyridone and/or one or more salts thereof in amounts by weight of 0.1% to 20%, preferably 1.1% to 10%, more preferably 1.5% to 7%, and with particular preference 5%,
b) one or more alcohols selected from 2-phenoxyethanol, 1-phenoxy-2-propanol, and benzyl alcohol, in amounts by weight of 35% to 99.8% and preferably of 59.8% to 99.8%, and
c) water in amounts by weight of 0.1% to 35%, preferably 0.1% to 20%, more preferably 0.5% to 5%, and with particular preference 1% to 3%.

4. The preservative composition of one or more of claims 1 to 3, **characterized in that** it comprises the aminoethanol salt of 1-hydroxy-4-methyl-6-(2,4,4-trimethylpentyl)-2(1H)-pyridone.

5. The preservative composition of one or more of claims 1 to 4, **characterized in that** the alcohol of component b) is 2-phenoxyethanol.

6. The preservative composition of one or more of claims 1 to 5, **characterized in that** it comprises one or more further active antimicrobial substances.

7. The preservative composition of claim 6, **characterized in that** it comprises the one or more further active antimicrobial substances in an amount of 1% to 25% by weight.

8. The preservative composition of claim 6 or 7, **characterized in that** the one or more further active antimicrobial substances are selected from parabens, preferably methylparaben, ethylparaben, propylparaben, butylparaben, isobutylparaben, sodium methylparaben, sodium ethylparaben, sodium propylparaben, sodium isobutylparaben and/or sodium butylparaben, organic acids, preferably sorbic acid, potassium sorbate, salicylic acid, sodium salicylate, benzoic acid and/or sodium benzoate, formaldehyde donors, preferably imidazolidinylurea, diazolidinylurea, DMDM hydantoin and/or sodium hydroxymethylglycinate, and halogenated preservatives, preferably iodopropynyl butylcarbamate and/or 2-bromo-2-nitropropane-1,3-diol.

9. The preservative composition of claim 8,
**characterized in that** it comprises
I) aminoethanol salt of 1-hydroxy-4-methyl-6-(2,4,4-trimethylpentyl)-2(1H)-pyridone, 2-phenoxyethanol, and, methylparaben or
II) aminoethanol salt of 1-hydroxy-4-methyl-6-(2,4,4-trimethylpentyl)-2(1H)-pyridone, 2-phenoxyethanol, and sodium benzoate or
III) aminoethanol salt of 1-hydroxy-4-methyl-6-(2,4,4-trimethylpentyl)-2(1H)-pyridone, 2-phenoxyethanol, and benzoic acid or
IV) aminoethanol salt of 1-hydroxy-4-methyl-6-(2,4,4-trimethylpentyl)-2(1H)-pyridone, 2-phenoxyethanol, and potassium sorbate or
V) aminoethanol salt of 1-hydroxy-4-methyl-6-(2,4,4-trimethylpentyl)-2(1H)-pyridone, 2-phenoxyethanol, and sorbic acid or
VI) aminoethanol salt of 1-hydroxy-4-methyl-6-(2,4,4-trimethylpentyl)-2(1H)-pyridone, 2-phenoxyethanol, and sodium salicylate or
VII) aminoethanol salt of 1-hydroxy-4-methyl-6-(2,4,4-trimethylpentyl)-2(1H)-pyridone, 2-phenoxyethanol, and salicylic acid.

10. The preservative composition of one or more of claims 1 to 9, **characterized in that** it comprises one or more antioxidants.

11. The preservative composition of claim 10, **characterized in that** the one or more antioxidants are selected from tocopheryl acetate, BHT, and EDTA.

12. The preservative composition of one or more of claims 1 to 11, **characterized in that** it comprises one or more hydrotropes.

13. The preservative composition of claim 12,
**characterized in that** the hydrotrope is cumenesulfonate.

14. The preservative composition of one or more of claims 1 to 13, **characterized in that** it comprises one or more solubilizers.

15. The preservative composition of claim 14, **characterized in that** the one or more solubilizers are selected from triethylene glycol, butylene glycol, and 1,2-propylene glycol.

16. The preservative composition of one or more of claims 1, 4, and 5, **characterized in that** it is composed of
a) 1-hydroxy-4-methyl-6-(2,4,4-trimethylpentyl)-2(1H)-pyridone and/or one or more salts thereof and
b) one or more alcohols selected from 2-phenoxyethanol, 1-phenoxy-2-propanol, and benzyl alcohol.

17. The preservative composition of one or more of claims 1 to 5, **characterized in that** it is composed of
a) 1-hydroxy-4-methyl-6-(2,4,4-trimethylpentyl)-2(1H)-pyridone and/or one or more salts thereof,
b) one or more alcohols selected from 2-phenoxyethanol, 1-phenoxy-2-propanol, and benzyl alcohol, and
c) water.

18. The use of a composition of one or more of claims 1 to 17 for preserving dermatological, cosmetic, and pharmaceutical products.

19. The use of a composition of one or more of claims 1 to 17 for preserving wet wipes.

20. The use of a composition of one or more of claims 1 to 17 for preserving emulsions.

21. The use of a composition of one or more of claims 1 to 17 for preparing a cosmetic, dermatological or pharmaceutical preparation.

22. A cosmetic, dermatological or pharmaceutical formulation comprising
b) aminoethanol salt of 1-hydroxy-4-methyl-6-(2,4,4-trimethylpentyl)-2(1H)-pyridone,
b) 2-phenoxyethanol and
c) one or more substances selected from benzoic acid, sodium benzoate, sorbic acid, potassium sorbate, salicylic acid, and sodium salicylate.

23. The formulation of claim 22, **characterized in that** the substances of components a), b), and c), based on the completed formulation, are present in the formulation at 0.1% to 2% by weight.

24. The formulation of claim 22 or 23, **characterized in that** it is an emulsion.

25. The formulation of claim 22 or 23, **characterized in that** it is a surfactant-free emulsion.

## Revendications

1. Agent conservateur liquide transparent, comprenant :
a) de la 1-hydroxy-4-méthyl-6-(2,4,4-triméthylpentyl)-2(1H)-pyridone et/ou un ou plusieurs sels de celle-ci dans des quantités pondérales allant de 0,1% à 20%, de préférence de 1,1% à 10%, de manière plus particulièrement préférée de 1,5 à 7% et de manière tout particulièrement préférée de 5% et
b) un ou plusieurs alcools choisis dans le groupe constitué par le 2-phénoxyéthanol, le 1-phénoxy-2-propanol et l'alcool benzylique dans des quantités pondérales allant de 35% à 99,9% et de préférence de 80% à 99,9%.

2. Agent conservateur selon la revendication 1, **caractérisé en ce qu'**il comprend :
a) de la 1-hydroxy-4-méthyl-6-(2,4,4-triméthylpentyl)-2(1H)-pyridone et/ou un ou plusieurs sels de celle-ci,
b) un ou plusieurs alcools choisis dans le groupe constitué par le 2-phénoxyéthanol, le 1-phénoxy-2-propanol et l'alcool benzylique,
c) de l'eau,
d) éventuellement une ou plusieurs autres substances antimicrobiennes,
e) éventuellement un ou plusieurs agents hydrotropes, et
f) éventuellement un ou plusieurs autres additifs.

3. Agent conservateur selon la revendication 1 ou 2, **caractérisé en ce qu'**il comprend .
a) le 1-hydroxy-4-méthyl-6-(2,4,4-triméthylpentyl)-2 (1H)-pyridone et/ou un ou plusieurs sels de celle-ci dans des quantités pondérales allant de 0,1% à 20%, de préférence de 1,1% à 10%, de manière plus particulièrement préférée de 1,5% à 7% et de manière tout particulièrement préférée de 5%,
b) un ou plusieurs alcools choisis dans le groupe constitué par le 2-phénoxyéthanol, le 1-phénoxy-2-propanol et l'alcool benzylique dans des quantités pondérales allant de 35% à 99,8% et de préférence de 59,8% à 99,8% et
c) de l'eau dans des quantités pondérales allant de 0,1% à 35%, de préférence de 0,1% à 20%, de manière plus particulièrement préférée de 0,5% à 5% et de manière plus particulièrement préférée de 1% à 3%.

4. Agent conservateur selon l'une quelconque ou plusieurs des revendications 1 à 3, **caractérisé en ce qu'**il comprend le sel d'aminoéthanol de la 1-hydroxy-4-méthyl-6-(2,4,4-triméthylpentyl)-2(1H)-pyridone.

5. Agent conservateur selon l'une quelconque ou plusieurs des revendications 1 à 4, **caractérisé en ce que** l'alcool du composant b) est le 2-phénoxyéthanol.

6. Agent conservateur selon l'une quelconque ou plusieurs des revendications 1 à 5, **caractérisé en ce qu'**il comprend une ou plusieurs autres substances antimicrobiennes.

7. Agent conservateur selon la revendication 6, **caractérisé en ce qu'**il comprend une ou plusieurs autres substances antimicrobiennes à raison de 1% à 25%.

8. Agent conservateur selon la revendication 6 ou 7, **caractérisé en ce que** les une ou plusieurs autres substances antimicrobiennes sont choisies dans le groupe constitué par les parabènes, de préférence le méthylparabène, l'éthylparabène, le propylparabène, le butylparabène, l'isobutylparabène, le méthylparabène sodique, l'éthylparabène sodique, le propylparabène sodique, l'isobutylparabène sodique et/ou le butylparabène sodique, les acides organiques, de préférence l'acide sorbique, le sorbate de potassium, l'acide salicylique, le salicylate de sodium, l'acide benzoïque et/ou le benzoate de sodium, les produits libérateurs de formaldéhyde, de préférence l'imidazolidinylurée, la diazolidinylurée, la DMDM hydantoïne et/ou l'hydroxyméthylglycinate de sodium et des agents conservateurs halogénés, de préférence le butylcarbamate de iodopropynyle, et/ou le 2-bromo-2-nitropropane-1,3-diol.

9. Agent conservateur selon la revendication 8, **caractérisé en ce qu'**il comprend :
I) le sel d'aminoéthanol de la 1-hydroxy-4-méthyl-6-(2,4,4-triméthylpentyl)-2(1H)-pyridone, du 2-phénoxyéthanol et du méthylparabène ou
II) le sel d'aminoéthanol de la 1-hydroxy-4-méthyl-6-(2,4,4-triméthylpentyl)-2(1H)-pyridone, du 2-phénoxyéthanol et du benzoate de sodium ou
III) le sel d'aminoéthanol de la 1-hydroxy-4-méthyl-6-(2,4,4-triméthylpentyl)-2(1H)-pyridone, du 2-phénoxyéthanol et de l'acide benzoïque ou
IV) le sel d'aminoéthanol de la 1-hydroxy-4-méthyl-6-(2,4,4-triméthylpentyl)-2(1H)-pyridone, du 2-phénoxyéthanol et du sorbate de potassium ou
V) le sel d'aminoéthanol de la 1-hydroxy-4-méthyl-6-(2,4,4-triméthylpentyl)-2(1H)-pyridone, du 2-phénoxyéthanol et de l'acide sorbique ou
VI) le sel d'aminoéthanol de la 1-hydroxy-4-méthyl-6-(2,4,4-triméthylpentyl)-2(1H)-pyridone, du 2-phénoxyéthanol et du salicylate de sodium ou
VII) le sel d'aminoéthanol de la 1-hydroxy-4-méthyl-6-(2,4,4-triméthylpentyl)-2(1H)-pyridone, du 2-phénoxyéthanol et de l'acide salicylique.

10. Agent conservateur selon l'une quelconque ou plusieurs des revendications 1 à 9, **caractérisé en ce qu'**il comprend un ou plusieurs antioxydants.

11. Agent conservateur selon la revendication 10, **caractérisé en ce que** les un ou plusieurs antioxydants sont choisis dans le groupe constitué par l'acétate de tocophéryle, le BHT et l'EDTA.

12. Agent conservateur selon l'une quelconque ou plusieurs des revendications 1 à 11, **caractérisé en ce qu'**il comprend un ou plusieurs agents hydrotropes.

13. Agent conservateur selon la revendication 12, **caractérisé en ce que** l'agent hydrotrope est le cumènesulfonate.

14. Agent conservateur selon l'une quelconque ou plusieurs des revendications 1 à 13, **caractérisé en ce qu'**il comprend un ou plusieurs solubilisants.

15. Agent conservateur selon la revendication 14, **caractérisé en ce que** les un ou plusieurs solubilisants sont choisis dans le groupe constitué par le triéthylèneglycol, le butylèneglycol et le 1,2-propylèneglycol.

16. Agent conservateur selon l'une quelconque ou plusieurs des revendications 1, 4 et 5, **caractérisé en ce qu'**il se compose de :
a) 1-hydroxy-4-méthyl-6-(2,4,4-triméthylpentyl)-2(1H)-pyridone et/ou d'un ou plusieurs sels de celle-ci et
b) un ou plusieurs alcools choisis dans le groupe constitué par le 2-phénoxyéthanol, le 1-phénoxy-2-propanol et l'alcool benzylique.

17. Agent conservateur selon l'une quelconque ou plusieurs des revendications 1 à 5, **caractérisé en ce qu'**il se compose de:
a) 1-hydroxy-4-méthyl-6-(2,4,4-triméthylpentyl)-2(1H)-pyridone et/ou d'un ou plusieurs sels de celui-ci,
b) un ou plusieurs alcools choisis dans le groupe constitué par le 2-phénoxyéthanol, le 1-phénoxy-2-propanol et l'alcool benzylique, et
c) eau.

18. Utilisation d'un agent selon l'une quelconque ou plusieurs des revendications 1 à 17 pour la conservation de produits pharmaceutiques, cosmétiques et dermatologiques.

19. Utilisation d'un agent selon l'une quelconque ou plusieurs des revendications 1 à 17 pour la conservation de lingettes humides.

20. Utilisation d'un agent selon l'une quelconque ou plusieurs des revendications 1 à 17 pour la conservation d'émulsions.

21. Utilisation d'un agent selon l'une quelconque ou plusieurs des revendications 1 à 17 pour la fabrication d'une préparation pharmaceutique, dermatologique ou cosmétique.

22. Préparation pharmaceutique, dermatologique ou cosmétique comprenant :
a) le sel d'aminoéthanol de la 1-hydroxy-4-méthyl-6-(2,4,4-triméthylpentyl)-2(1H)-pyridone,
b) du 2-phénoxyéthanol et
c) une ou plusieurs substances choisies dans le groupe constitué par l'acide benzoïque, le benzoate de sodium, l'acide sorbique, le sorbate de potassium, l'acide salicylique et le salicylate de sodium.

23. Préparation selon la revendication 22, **caractérisée en ce que** les substances des composants a), b) et c) sont contenues, par rapport aux préparations finies, à raison de 0,1% à 2% en poids dans les préparations.

24. Préparation selon la revendication 22 ou 23, **caractérisée en ce qu'**il s'agit d'une émulsion.

25. Préparation selon la revendication 22 ou 23, **caractérisée en ce qu'**il s'agit d'une émulsion exempte de tensio-actifs.
